# EUROPEAN PATENT SPECIFICATION

(11) **EP 2 575 604 B1**
(45) Date of publication and mention of the grant of the patent: **05.07.2023**
(21) Application number: 11787530.2
(22) Date of filing: 27.05.2011
(51) Int. Cl.: A61B 5/02, A61B 8/12, A61B 5/07, A61B 5/00, A61B 5/1455, A61B 5/0205

(54) **APPARATUS, SYSTEMS, METHODS AND COMPUTER-ACCESSIBLE MEDIUM FOR ANALYZING INFORMATION REGARDING CARDIOVASCULAR DISEASE(S) AND FUNCTION(S)**
VORRICHTUNG, SYSTEME, VERFAHREN UND COMPUTERLESBARES MEDIUM ZUR ANALYSE VON INFORMATIONEN ÜBER HERZ-KREISLAUFKRANKHEITEN UND -FUNKTIONEN
APPAREIL, SYSTÈMES, PROCÉDÉS ET SUPPORT INFORMATIQUE POUR L'ANALYSE D'INFORMATION CONCERNANT UNE OU PLUSIEURS AFFECTIONS ET FONCTIONS CARIDOVASCULAIRES

(30) Priority: 28.05.2010 US 349579 P
(43) Date of publication of application: 10.04.2013
(73) Proprietor: The General Hospital Corporation, Boston, MA 02114 (US)
(72) Inventor: SUBRAMANIAM, Balachundhar, Lexington MA 02421 (US); WARGER, William, C.II, Lexington MA 02421 (US); SIMON, Brett, Lexington MA 02421 (US); TEARNEY, Guillermo, J., Cambridge MA 02139 (US); LI, Li, MA 02169 (US)
(74) Representative: Maiwald GmbH
(86) International application number: PCT/US2011/038421
(87) International publication number: WO 2011/150379

(56) References cited:
- WO-A1-2009/001913
- WO-A1-2009/076427
- WO-A1-2010/009747
- WO-A2-2004/091361
- IL-A- 150 167
- US-A- 5 840 031
- US-A1- 2005 215 911
- US-A1- 2006 106 375
- US-A1- 2006 167 363
- US-A1- 2006 224 053
- US-A1- 2007 010 709
- US-A1- 2007 260 146
- US-A1- 2009 124 853
- US-A1- 2009 177 033
- US-A1- 2010 121 315

## Description

### CROSS-REFERENCE TO RELATED APPLICATION(S)

The present application is based on and claims priority from U.S. Patent Application Serial No. 61/349,579, filed on May 28, 2010.

### FIELD OF THE DISCLOSURE

Exemplary embodiments of the present disclosure relates generally to apparatus, systems, methods and computer accessible medium for analyzing information regarding cardiovascular diseases and functions, and more particular apparatus, systems, methods and computer accessible medium analyzing for at least one characteristic of an anatomical structure, and even more particularly to the utilization of at least one electromagnetic or acoustic wave to diagnose cardiovascular diseases and/or measure cardiac functions.

### BACKGROUND INFORMATION

For example, a coronary artery disease, mainly caused by atherosclerosis, is the leading cause of death worldwide. Such disease occurs when fats, lipid, calcium, collagen, macrophage and other substances form a plaque on the walls of a artery. There may exist a group of "vulnerable plaques" that can be characterized by the anatomy and composition. These vulnerable plaques tend to rupture, which can cause either a myocardial infarction or a stroke, and can ultimately lead to death. Preferably, it is better to identify those in the population who have these vulnerable plaques, so that that they may be treated and heart attack may be prevented.

Current diagnostic methods and/or procedures for coronary artery diseases have certain limitations that can make it challenging to screen for the coronary artery disease or vulnerable plaques. Coronary angiography, a conventionally preferred procedure that uses an invasive cardiac catheterization, is costly, and has a finite risk of serious complications. Because this procedure visualizes the lumen, and not the wall of a blood vessel, it tends to underestimate the amount and extent of atherosclerosis. Finally, this procedure does not provide information about the composition of the plaques. Intravascular ultrasound (IVUS) and intravascular optical coherence tomography ("OCT" - "IVOCT") procedures are likely more capable of imaging the artery wall structure and can characterize plaque, but are similarly invasive, and therefore not ideal for screening. Computed tomography ("CT") and magnetic resonance imaging ("MRI") have been described as non-invasive alternatives for diagnosing coronary artery diseases. These techniques are capable of imaging the bulk structure of the coronary artery wall, but are relatively expensive, use contrast administration, which are features that may not be ideal for screening large populations for coronary artery disease and vulnerable plaque.

In addition, it is important to provide a reliable means for measuring cardiac functions during all key physiological states. E.g. mixed venous oxygen saturation, blood oxygenation measured from the pulmonary artery, can be a beneficial option for assessing perfusion as it can provide a continuous, real time measure of cardiac output, arterial oxygen saturation, and global oxygen extraction. Mixed venous oxygen saturation less than 65% has been associated with poor surgical outcomes (see, e.g., Jhanji, S., et al. "Microvascular flow and tissue oxygenation after major abdominal surgery: association with post-operative complications", Intensive Care Medicine 35, 671-677 (2009)), with a majority of these complications occurring during a low cardiorespiratory reserve, where pulse oximetry is ineffective. A continuous display of a mixed venous oxygen saturation can be possible with oximetric Swan Ganz catheters, inserted transcutaneously through the jugular vein into the pulmonary artery. These catheters are generally invasive, -and can cause substantial added morbidity and mortality to critically ill patients. As a result, continuous monitoring of cardiac functions, such as blood oxygenation in a major blood vessel attached to the heart in acute patients, can be a significant challenge, especially for those with low blood volume following trauma (e.g., traffic accidents or combat situations), shock (e.g., sepsis, hypovolemia or cardiogenic) and burns.

Pulse oximetry procedures can measure oxygen saturation from the peripheral arteries, such as fingers and earlobes, and can provide an approximate quantity of oxygen supplied to the organs. This procedure has been beneficially used for a clinical care of patients in emergency rooms, medical and surgical floors, operating rooms, and intensive care units, as it assist with the detection of patients prone to hypoxic events. (See, e.g., Shelley, K. H., "Photoplethysmography: Beyond the Calculation of Arterial Oxygen Saturation and Heart Rate", Anesthesia and Analgesia 105, S31-S36 (2007)). However, this technique relies on a consistent perfusion of all peripheral arteries, and the inaccuracy likely increases with a decreased oxygen saturation. US 2010/0174197 A1 and WO2009/076427 A1 relate to photoacoustic imaging devices.

Thus, it may be beneficial to address and/or overcome at least some of the deficiencies of the prior approaches, procedures and/or systems that have been described herein above.

### OBJECTS AND SUMMARY OF EXEMPLARY EMBODIMENTS

It is therefore one of the objects of the present disclosure to reduce or address the deficiencies and/or limitations of such prior art approaches, procedures, methods, systems, apparatus and computer-accessible medium.

For example, according to one object of the exemplary embodiments of the present disclosure is to provide apparatus, systems, methods and computer accessible medium for diagnosing cardiovascular disease and vulnerable plaque. Another object of another exemplary embodiment of the present disclosure can be to provide apparatus, systems, methods and computer accessible medium for measuring cardiac functions during most or all key physiologic states. The current invention is defined in independent claim 1. Preferred embodiments of the current invention are defined in the dependent claims.

Thus, according to exemplary embodiments of the present disclosure, method and apparatus can be provided, in which a first (e.g., excitation) radiation can be used to illuminate a tissue, and a second (e.g., emission) radiation can be generate as a result of an interaction between the tissue and the first radiation. For example, the second radiation can be measured, a measurement as a dataset can be made, and the dataset can be analyzed to determine at least one characteristic of the tissue.

According to one exemplary embodiment of the present disclosure, a source for providing the excitation radiation and/or a detector to measure the emission radiation can be placed outside the tissue. The radiation can be an electromagnetic wave and/or an acoustic wave. The tissue can be a part of a heart, including, e.g., a left or a right ventricle, a left or right atrium, a myocardium, a mitral valve, a tricuspid valve, an aortic valve, a pulmonary valve, a pericardium, or a pericardial fluid, etc. The tissue can also be a blood vessel attached to a heart, including, e.g., an aorta, a pulmonary artery, a pulmonary vein, an inferior vena cava, a superior vena cava, a coronary artery, and/or a coronary vein, etc. The tissue can further be blood in a blood vessel, and/or an atherosclerotic plaque in a blood vessel or a heart. The measurable characteristic of the tissue can include, but is not limited to, an anatomy or composition of an atherosclerotic plaque, or an oxygen saturation level of a blood, etc.

In one exemplary embodiment of the present disclosure, apparatus and method can be provided for determining information regarding a tissue or an object at or within the tissue. For example, with at least one first arrangement which is situated inside a particular organ of the body, it is possible to generate at least one electromagnetic radiation in the tissue, wherein the tissue is different from and outside of the particular organ. The particular signals that are responsive to the at least one electromagnetic radiation can be detected (e.g., possibly with at least one second arrangement), at least one characteristic of the tissue and/or information regarding the object at or in the tissue can be determined (e.g., with the second arrangement(s)).. Alternatively or in addition, the tissue can be different from and outside of the particular organ, and the first arrangement(s) can be situated inside a particular organ of the body. To that end, the tissue can include (i) the heart, (ii) major vessels attached to the heart, (iii) coronary artery, and/or (iv) blood therein. Alternatively or in addition, the tissue can be different from and outside of the particular organ, and the first arrangement(s) can be situated inside a particular organ of the body. To that end, the tissue can include (i) the heart, (ii) major vessels attached to the heart, (iii) coronary artery, and/or (iv) blood therein

For example, the characteristic(s) can include (i) blood, (ii) one or more major blood vessels coupled to a heart, and/or (iii) one or more portions of the heart. The particular organ can be an esophagus. The second arrangement(s) can be further configured to determine further information to identify at least a possibility at least one coronary artery disease. The first and/or second arrangement(s) can be structured and sized to be at least partially situated within the esophagus. The first and/or second arrangement can be (i) photoacoustic arrangement, (ii) a fluorescence arrangement, (iii) an optical spectroscopy arrangement, (iv) a laser speckle imaging arrangement, (v) an optical tomography arrangement, and/or (vi) an ultrasound arrangement. The first and/or second arrangement(s) can be included with or in a transnasal device.

According a yet another exemplary embodiment of the present disclosure, at least one third arrangement can be provided which is configured to generate at least one image of the tissue as a function of the information. The third arrangement(s) can be configured to obtain data for (i) a structure of the tissue, and (ii) the characteristic(s) approximately simultaneously. The data can include at least one image of (i) the structure of the tissue, and/or (ii) the characteristic(s) superimposed on one another. The second arrangement(s) can further measure at least one characteristic of at least one further tissue which includes one or more other tissues in addition to the one or more major blood vessel or portion of the heart. At least one fourth arrangement can also be provided which is configured to generate at least one image of the tissue and a further tissue that is in a proximity of the tissue. Such fourth arrangement(s) can include an ultrasound arrangement.

[[According to a further exemplary embodiment of the present disclosure, apparatus and method can be provided for determining at least one characteristic of an anatomical structure. For example, with at least one first photo-acoustic arrangement, a generation of an acoustic wave can be caused in the anatomical structure. The acoustic wave can be detected, and (possibly with at least one second arrangement), at least one characteristic of blood can be measured within the anatomical structure which is (i) one or more of major blood vessels coupled to a heart, and/or (ii) one or more portions of the heart. The measurement can be performed outside of the anatomical structure.

For example, the first arrangement(s) and/or the second arrangement(s) can be structured and sized to be at least partially situated within an esophagus. The characteristic(s) of the anatomical structure can include an oxygen saturation, a cardiac output, a blood flow, a total blood content and/or a blood hematocrit. The oxygen saturation characteristic(s) can include a venous oxygen saturation and/or a arterial oxygen saturation. In addition, the first arrangement(s) and/or the second arrangement(s) can be included with a transnasal device. Further, it is possible to generate at least one image of the anatomical structure as a function of the acoustic wave, e.g., using at least one third arrangement. It is possible to obtain data for (i) a structure of the anatomical structure, and (ii) the at least one characteristic approximately simultaneously, e.g., with the third arrangement(s). Such exemplary data can include at least one image of: (i) the structure of the anatomical structure, and/or (ii) the characteristic(s) of the anatomical structure superimposed on one another.

These and other objects, features and advantages of the exemplary embodiments of the present disclosure will become apparent upon reading the following detailed description of the exemplary embodiments of the present disclosure, when taken in conjunction with the appended claims.

### BRIEF DESCRIPTION OF THE DRAWINGS

Further objects, features and advantages of the present disclosure will become apparent from the following detailed description taken in conjunction with the accompanying figures showing illustrative embodiments of the present disclosure, in which:
Figure 1 is a diagram of a wireless transesophageal pill endoscope arrangement/apparatus according an exemplary embodiment of the present disclosure, which can access the cardiovascular diseases and functions;
Figure 2(a) is a diagram of the transesophageal pill endoscope on a string according another exemplary embodiment of the present disclosure, which can access the cardiovascular diseases and functions;
Figure 2(b) is a diagram of the transesophageal pill endoscope on a steering string according still another exemplary embodiment of the present disclosure;
Figure 3(a) is a diagram of an apparatus according an exemplary embodiment of the present disclosure which includes an inflatable balloon;
[0011] Figure 3(b) is a diagram of the apparatus according an exemplary embodiment of the present disclosure which includes a guide wire;
Figure 4(a) is an illustration of an exemplary method which introduce the apparatus (including the pill) according an exemplary embodiment of the present disclosure into a subject;
Figure 4(b) is an illustration of an exemplary method which introduce the apparatus (including the transnasal pill) according another exemplary embodiment of the present disclosure into a subject;

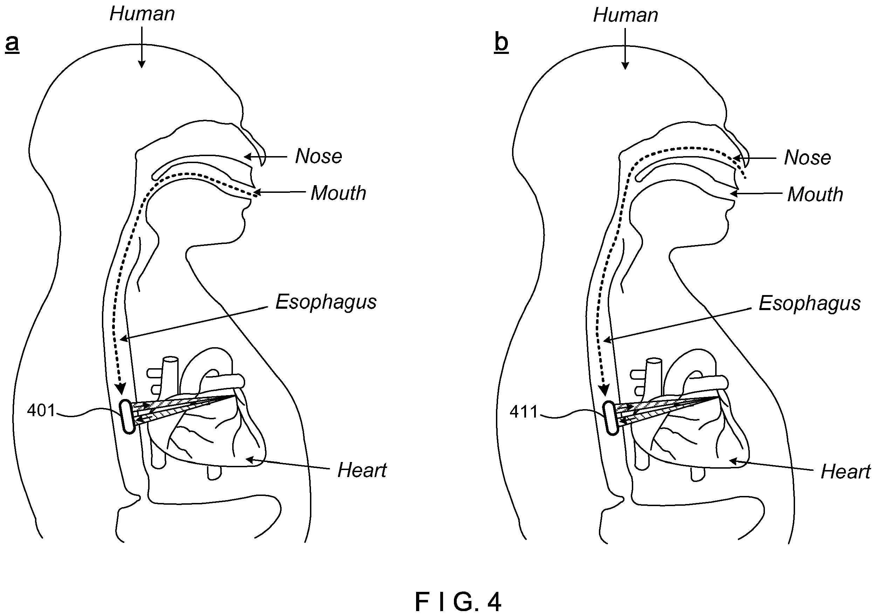

Figure 4(b) is an illustration of an exemplary method which introduce the apparatus (including the transnasal tube) according still another exemplary embodiment of the present disclosure into a subject;

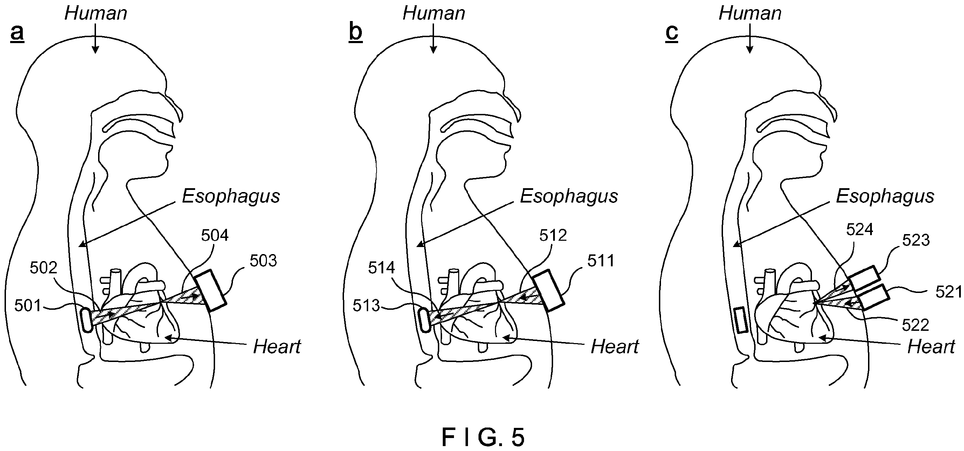

Figure 5(a) is an illustration of an exemplary configuration of the apparatus according a further exemplary embodiment of the present disclosure, where an exemplary transesophageal pill endoscope can provide an excitation radiation, and an emission radiation can be detected by a detector placed outside of a subject;
Figure 5(b) is an illustration of an exemplary configuration of the apparatus according yet another exemplary embodiment of the present disclosure, where a source outside of a subject provides an excitation radiation, and an emission radiation can be detected by the exemplary transesophageal pill endoscope;
Figure 5(c) is an illustration of an exemplary configuration of the apparatus according still further exemplary embodiment of the present disclosure, where a source outside of the subject provides an excitation radiation, and an emission radiation can be detected by an exemplary detector placed outside of a subject;

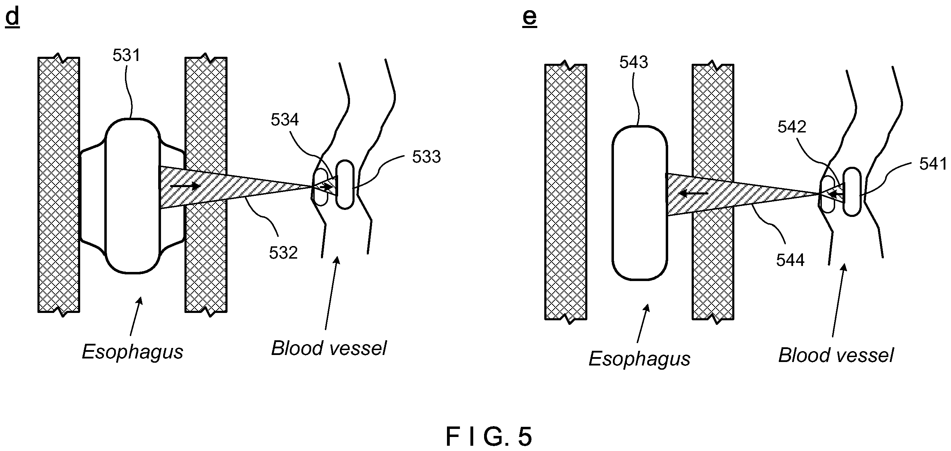

Figure 5(d) is an illustration of a configuration of another apparatus according to according an exemplary embodiment of the present disclosure, where the transesophageal pill endoscope provides an excitation radiation, and an emission radiation can be detected by the exemplary detector located inside a heart and/or a blood vessel;
Figure 5(e) is an illustration of a configuration of a further apparatus according to according an exemplary embodiment of the present disclosure, where a source located inside a heart or a blood vessel provides an excitation radiation, and an emission radiation can be detected by the transesophageal pill endoscope;

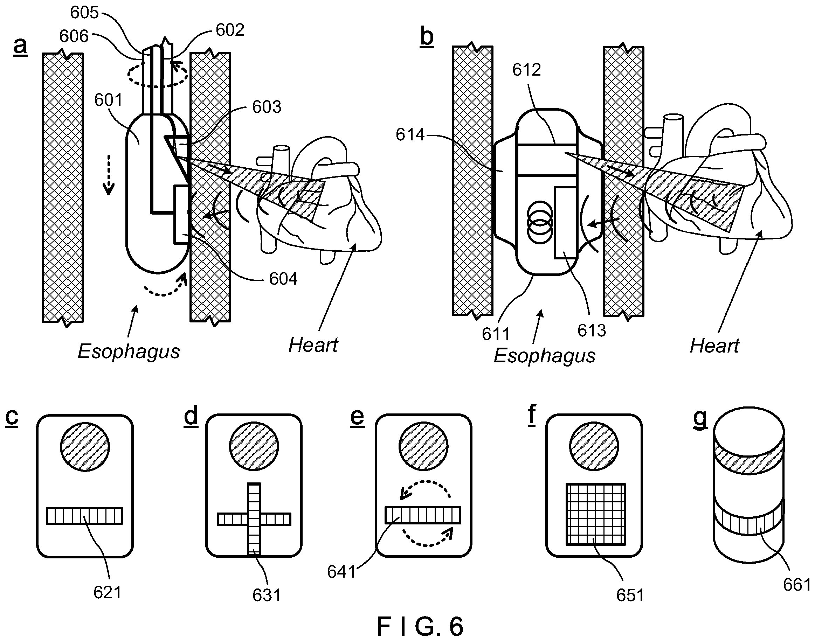

Figure 6(a)-6(g) are exemplary illustration of an exemplary configuration of still another apparatus according a further exemplary embodiment of the present disclosure, which is configured to have a photoacoustic set-up configuration;

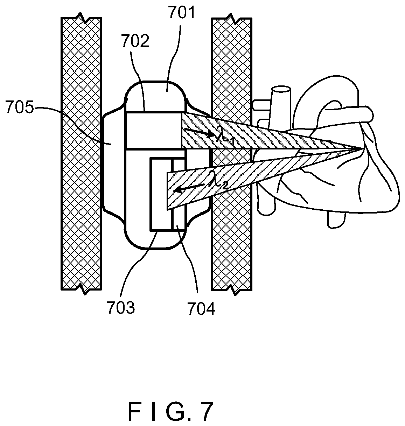

Figure 7 is an illustration of an exemplary configuration of a further apparatus according a still further exemplary embodiment of the present disclosure, which is configured to have a fluorescence imaging set-up configuration;

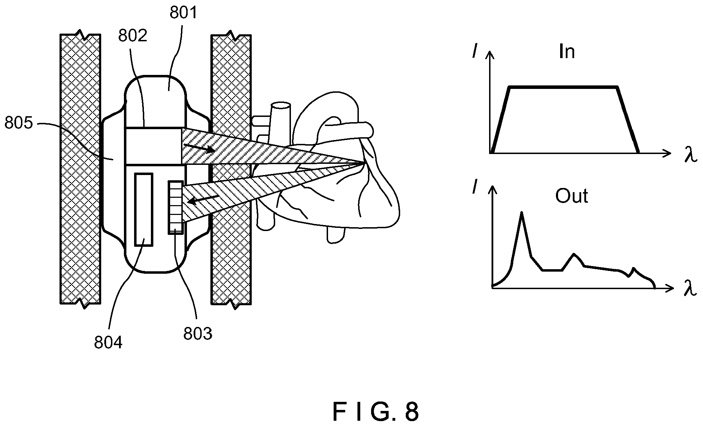

Figure 8 is an exemplary configuration of a further apparatus according yet another exemplary embodiment of the present disclosure, which is configured to have an optical spectroscopy set-up configuration, with associated plots provided in the illustration;

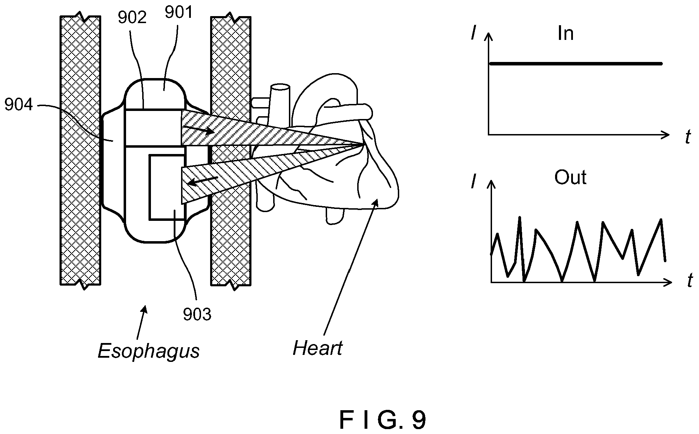

Figure 9 is an exemplary configuration of another apparatus according still another exemplary embodiment of the present disclosure, which is configured to have a laser speckle imaging set-up configuration, with associated plots provided in the illustration;

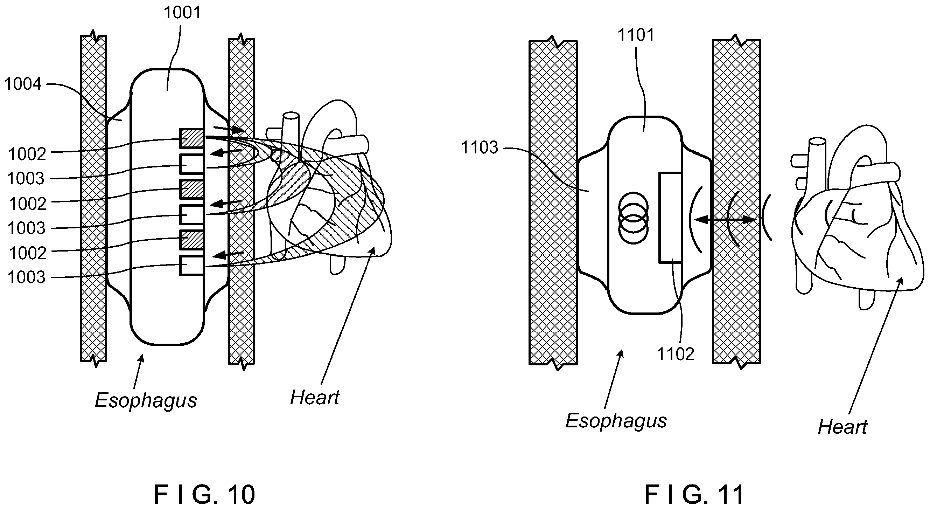

Figure 10 is an exemplary configuration of a further apparatus according yet another exemplary embodiment of the present disclosure, which is configured to have an optical tomography set-up configuration; and
Figure 11 is an exemplary configuration of a still further apparatus according a further exemplary embodiment of the present disclosure, which is configured to have an ultrasound imaging set-up configuration.

Throughout the figures, the same reference numerals and characters, unless otherwise stated, are used to denote like features, elements, components or portions of the illustrated embodiments. Moreover, while the subject disclosure will now be described in detail with reference to the figures, it is done so in connection with the illustrative embodiments. It is intended that changes and modifications can be made to the described exemplary embodiments without departing from the true scope of the subject disclosure as defined by the appended claims.

### DETAILED DESCRIPTION OF EXEMPLARY EMBODIMENTS

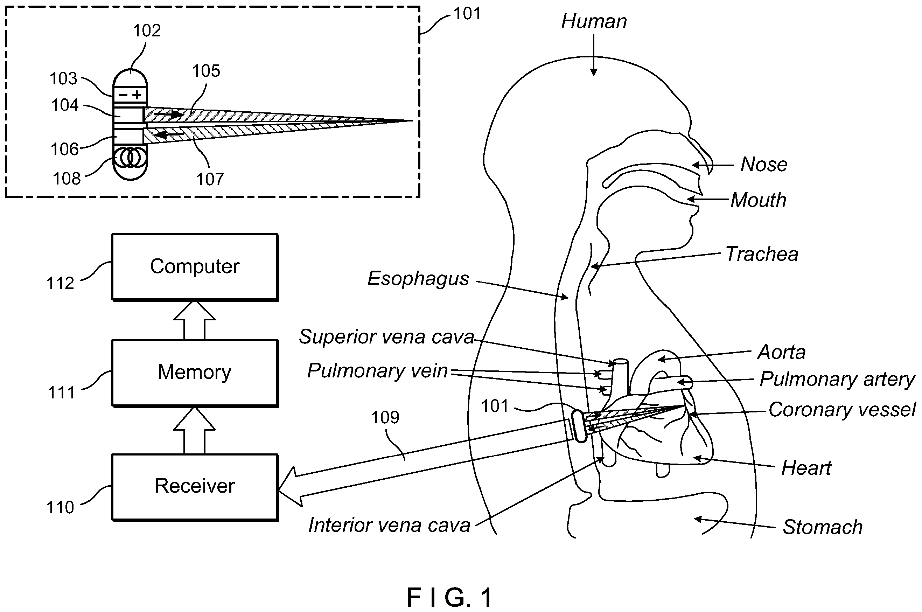

An exemplary embodiment of method and apparatus according to an exemplary embodiment of the present disclosure is illustrated in Figure 1. As shown in Figure 1, a pill endoscope 101, sized to fit inside an esophagus, can be provided, which can comprise a biocompatible housing 102, a power unit 103 (e.g., a battery), a source 104 (e.g., an electromagnetic radiation source) that can provide a first radiation 105 to excite a tissue, a detector 106 that can transfer a second radiation 107 emitted from the tissue to a first signal (e.g., an electric voltage), a transmitter 108 (e.g., a radio-frequency transmitter) that can convert the first signal into a second signal 109 (e.g., a radio-frequency electromagnetic wave). The second signal 109 can be acquired by a receiver 110 (e.g., an antenna), stored as a dataset on a memory 111 or in another storage device (e.g., RAM, ROM, hard drive, etc.), and analyzed by one or more computers 112 to provide at least one characteristic of the tissue.

The pill endoscope 101 can further include a sensor to measure a motion of an esophageal tissue, a temperature, a PH value and/or a pressure, etc. These exemplary measurements can be used to determine the current position of the pill endoscope 101 with and/or without operator intervention. The pill endoscope 101 can be carried by peristalsis through a digestive tract, and can also move independently from peristalsis via exemplary components for a magnetic steering, active propelling and/or robotic movement. The receiver 110 and the memory/storage device 111 can be made into a portable device, and carried by a subject during data acquisition. Alternatively or in addition, the pill endoscope 101 can have an onboard memory/storage to store the dataset; thus making the transmitter 108 unnecessary. The pill endoscope 101 can also include components providing facilitating an intervention, such as biopsy, treatment, etc. The measured characteristics of the tissue can further be used to guide the intervention by providing localization information and/or assisting in a selection of a personalized treatment.

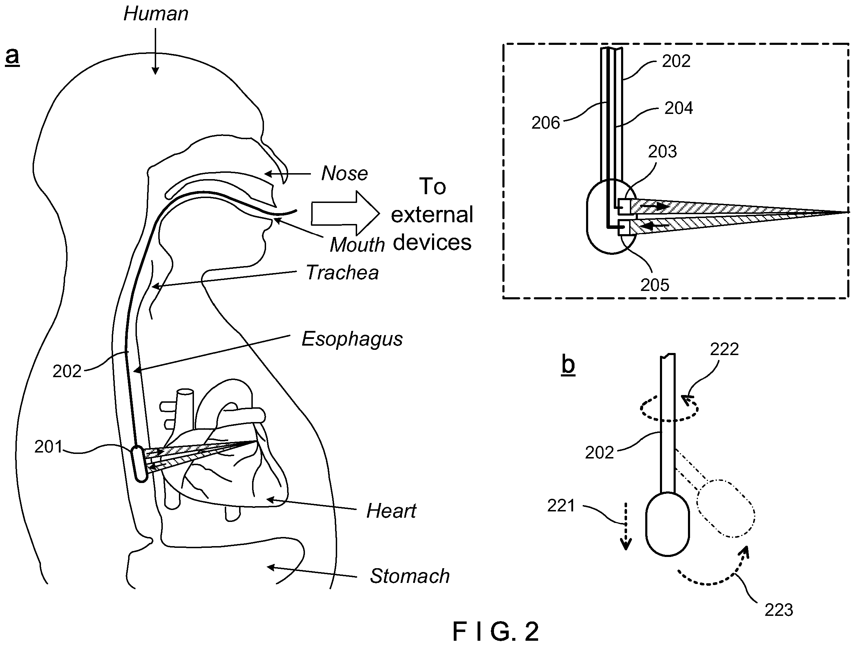

Another exemplary embodiment of the method and apparatus according to a further exemplary embodiment of the present disclosure is illustrated in Figure 2(a). For example, a transesophageal pill endoscope 201 of this exemplary embodiment can include a string 202. The string 202 can comprise a wire 204 that can deliver power and/or a first radiation to an excitation source 203, and/or a second wire 206 that can provide a signal generated by an emission detector 205 to an external receiver. Figure 2(b) shows a detailed illustration of the transesophageal pill endoscope 201 in which the string 202 can further be used to steer the pill endoscope 201 through translation 221, rotation 222 and/or flex 223, e.g., by using controls placed on the proximal end. After data acquisition, the pill endoscope 201 can also be retrieved by pulling the string 202.

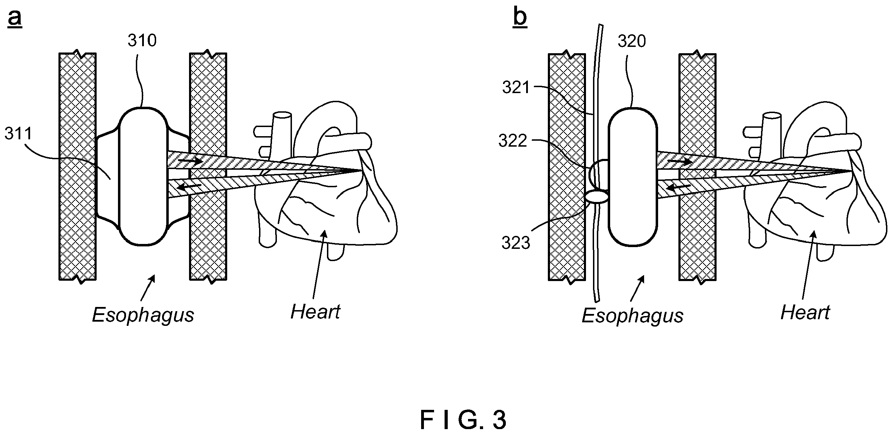

As illustrated in Figure 3(a), a transesophageal pill endoscope 310 according to another exemplary embodiment of the present disclosure can also include an inflatable balloon 311. For example, when the exemplary pill endoscope 310 reaches a particular position to measure a tissue of interest, the balloon 311 can be inflated (e.g., by providing an inflation medium via an inflation arrangement) to anchor the pill endoscope 310 in position, thus facilitating a continuous measurement of the tissue for a prolonged time period. The inflating medium can include air, water, deuterized water, saline, etc. In certain exemplary embodiments involving acoustic excitation and/or emission, an inflating medium (e.g., water) can further provide a proper acoustic coupling between the detector and the esophageal wall to minimize signal loss.

Figure 3(b) shows another transesophageal pill endoscope 320 according to a further exemplary embodiment of the present disclosure that can operate with a guide wire 321. For example, due to a small diameter of the guide wire 321, it can be first inserted into an esophagus relatively easily. The pill endoscope 320 can have an attachment 322 that can hook itself to the guide wire 321. An introduction of the pill endoscope 320 using the guide wire 321 can avoid unwanted rolling. The guide wire 321 can further include a stopper 323, which can stop the pill endoscope 320 at one or more particular locations to measure a tissue of interest.

Figures 4(a) and 4(b) illustrates two possible ways, respectively, to introduce the pill endoscope into an esophagus. For example, Figure 4a shows a transesophageal pill endoscope 401 according yet another exemplary embodiment of the present disclosure that can be introduced through the mouth, and swallowed into the esophagus. Figure 4(b) illustrates another transesophageal pill endoscope 411 according still another exemplary embodiment of the present disclosure that can be introduced alternatively through a nasal orifice via a nasopharynx into the esophagus. The trans-nasal introduction can be useful for placing the pill endoscope 411 with a string. Although such introduction of the pill endoscope 411 is performed via a relatively smaller diameter, it can be tolerated better by awake or mildly sedated subjects, and is can be suitable for continuous assessment of cardiac functions of a subject in an intensive care unit.

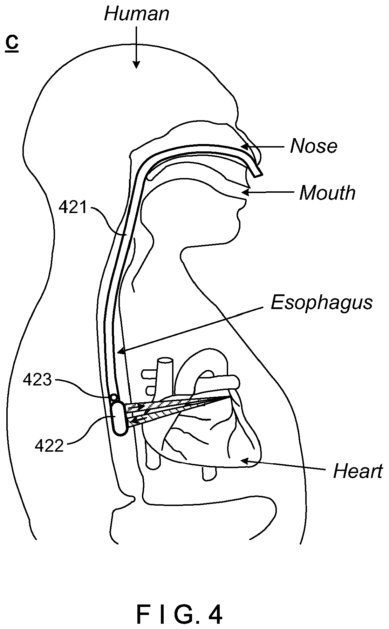

Alternatively or in addition, according to yet another exemplary embodiment of the present disclosure shown in Figure 4(c), the transnasal device can include a catheter or tube 421 that can be similarly inserted through the nose, until the electromagnetic radiation transducing arrangement/apparatus 422 is located in the esophagus, e.g., adjacent to the heart. The tube can include a balloon that surrounds such portion of the device. In one or more exemplary embodiments described herein, a pressure transducer 423 can be placed near the distal end of the device to provide pressure measurements that can assist in the automatic placement of the active portion of the device within the esophagus, such that it is located near the cardiac structures of interest.

Because in a subject the esophagus is located in the close proximity of the heart, the exemplary transesophageal arrangements can provide a close-up view of cardiovascular diseases and functions. Further, a variety of alternative arrangements can also be provided, according to further exemplary embodiments of the present disclosure. For example, Figure 5(a) shows another arrangement according to still another exemplary embodiment of the present disclosure, where a source 501 inside an esophagus can generate an excitation radiation 502 to illuminate the tissue, and an emission radiation 504 from the tissue can be detected by a detector 504 placed outside of the subject (e.g., on the thorax). Figure 5(b) shows yet another arrangement according to a further exemplary embodiment of the present disclosure, where a source 511 placed outside of a subject can emit an excitation radiation 512, and a detector 513 inside an esophagus can detect an emission radiation 514. Figure 5(c) shows another arrangement according to a still further exemplary embodiment of the present disclosure, where both a source 521 and a detector 523 are placed outside of the subject.

Furthermore, one or more parts or sections of the exemplary arrangement can be incorporated into a catheter, which can be inserted into a heart or a blood vessel. The exemplary catheter can facilitate an intervention, such as biopsy or treatment. Then, the exemplary apparatus according to an exemplary embodiment of the present disclosure can guide the intervention to be performed at one or more particular locations, and/or to a preferred extent. Figure 5(d) illustrates another arrangement according to yet another exemplary embodiment of the present disclosure, where a source 531 inside an esophagus can provide an excitation radiation 532, and a detector 533 inside a blood vessel or a part of a heart can measure an emission radiation 534. Figure 5(e) shows a further arrangement according to a further exemplary embodiment of the present disclosure, where a source 541 is placed in a heart or a blood vessel can provide an excitation radiation 542, and a detector 543 in an esophagus can measure an emission radiation 544.

According to a still further exemplary embodiment of the present disclosure, the exemplary arrangement/apparatus can include a photoacoustic arrangement. Such exemplary photoacoustic arrangement can be used to illuminate the tissue using an electromagnetic wave and/or radiation that can vary in intensity as a function of time, and this arrangement can detect an acoustic wave (e.g., a photoacoustic wave) provided from the tissue through a thermoelastic coupling process/procedure after absorbing one or more portions of the electromagnetic energy. The resultant acoustic wave can be propagated through the tissue with an attenuation and scattering that is weaker than that of a light. Thus, the exemplary photoacoustic device/arrangement can be used to interrogate the tissue at a great depth (on the order of centimeters) with a high resolution (e.g., <1 mm).

It is also possible to selectively generate an acoustic emission from a specific constituent of the tissue, e.g., by utilizing electromagnetic excitation covering the spectral range where the target absorbs strongly. The amplitude and/or a temporal profile of the detected ultrasound wave can provide the location, shape and/or quantity of the absorbing tissue. For example, lipid, a major constituent of atherosclerotic plaques, can be mapped by the exemplary photoacoustic device with the near-infrared optical excitation (e.g., 900 - 1800 nm) to detect plaque, while hemoglobin in blood can be detected using light with visible to near-infrared wavelength (e.g., 500 - 1100 nm) to depict the lumen of a heart or a blood vessel. In one exemplary embodiment of the present disclosure, it can be preferable to detect lipid, or more specifically, cholesterol or cholesterol esters, within the coronary artery wall.

For example, cholesterol absorption peaks, including those, e.g., around 950 nm, 1205 nm, 1750 nm, can be targeted by the incident optical radiation to create absorption or photo-acoustic effects that can be detected by the exemplary arrangements according to certain exemplary embodiments of the present disclosure. In so doing, it is possible (e.g., using such exemplary arrangements) to provide a screening diagnosis of the presence or absence of vulnerable plaque. In addition, an injectable exogenous chromogenic contrast agent (e.g., indocyanine green) can be used to tag a tissue of interest, or may be preferentially localized by enhanced uptake and/or retention, allowing a characteristic of the tagged tissue, such as leaky vessels known to be common in the lipid core of vulnerable plaque, be obtained using excitation wavelength close to the absorption peak of the contrast agent (e.g., approximately 800 nm for indocyanine green).

Exemplary photoacoustic methods can be used for quantifying the optical, thermal, and mechanical properties of a tissue. According to the difference in one or several measurable properties, the spatial and/or temporal distribution of different tissue constituents can be obtained. For example, because the oxy-hemoglobin and deoxy-hemoglobin have distinct optical absorption spectra, a plurality of photo-acoustic signals from blood can be acquired using optical excitation at a plurality of excitation wavelengths, Then, the local concentrations of the oxy-hemoglobin (C_{HbO}) and deoxy-hemoglobin (C_{HbR}) can be calculated from these photo-acoustic measurements. As a result, we can further obtain important physiological parameters, such as a blood oxygen saturation level (C_{HbO}/(C_{HbO}+C_{HbR})), the total hemoglobin concentration (C_{HbO}+C_{HbR}) and the blood hematocrit. In contrast to the traditional pulse oximetry, photoacoustic measurement of blood oxygenation works in patients with/without pulsation and maintains consistent accuracy when measuring deoxygenated and oxygenated blood.

Following a similar exemplary process, the exemplary photo-acoustic measurements made using excitation at a plurality of visible for near-infrared spectral bands can be used to quantify the distribution of lipid, calcium and collagen in a blood vessel wall, to detect or characterize an atherosclerotic plaque. A photoacoustic device can further sense temperature in tissue to assess the macrophage activity in an atherosclerotic plaque, because the photo-acoustic amplitude increases as temperature increases. The velocity of the blood can also be measured by a photo-acoustic set-up using the Doppler principle. Thus, photoacoustic method can facilitate an assessment of to which extent a stenosis impedes oxygen delivery to the heart, by measuring the blood flow and oxygenation at different locations of the blood vessel. Since the dimension of a blood vessel, oxygenation and flow can be measured using photoacoustic methods, a photo-acoustic embodiment of the present disclosure can further measure a cardiac output, a cardiac index, the pulmonary oxygen uptake, oxygen delivery to different parts of a body, etc.

Figure 6(a) shows one exemplary photoacoustic arrangement according to another exemplary embodiment of the present disclosure, which is provided in the form of a transesophageal pill endoscope on a string. For example, light generated by a pulsed laser (e.g., a nanosecond Nd:YAG pumped OPO laser, tunable from 650 - 2500 nm, although other wavelengths are conceivable and are within the scope of the present disclosure) can be delivered to a pill arrangement 601 through a fused silica multimode optical fiber 602, directed by a micro-prism 603, to illuminate a tissue. A generated acoustic wave can be converted by an ultrasonic transducer 604 into an electric signal, which can be carried by an electric wire 605 to external devices. The signal may be amplified, digitized, and acquired using the ultrasonic transducer 604 to a computer that can further analyze the signal to provide at least one characteristic of the tissue. The exemplary pill arrangement 601 can be steered by an attached string 606 to come to a close contact with an esophageal wall to provide an appropriate acoustic coupling, and achieve a desirable view of the tissue.

Figure 6(b) illustrates another exemplary photoacoustic arrangement in the form of a wireless transesophageal pill endoscope according to a further exemplary embodiment of the present disclosure. For example, inside an exemplary pill arrangement 611, a miniature light source 612 (e.g., a microchip laser, a pulsed laser diode, a modulated continuous-wave laser diode or a fiber laser) powered by a battery or a remote power unit can be provided which can generate light to excite the tissue, and an ultrasonic transducer 613 can be provided which can detect a resultant acoustic wave, and transmit it to an external receiver. The signal can then be acquired to a computer, and analyzed to provide information about the tissue.

The pill arrangement 611 can include a balloon 614, which can be inflated with an acoustic coupling medium (e.g., water) to stop at one or more particular positions to detect, review or look at the tissue of interest. An ultrasonic transducer is a device that can sense an acoustic wave. This device can be a piezoelectric transducer, a polyvinylidene fluoride film transducer, a capacitor micro-machined transducer and/or any acoustic sensor based on an optical interferometer. The transducer can also include a single-element acoustic hydrophone or microphone, or an array of acoustic transducers. The ultrasonic detection array can have different configurations, including, but are not limited to, a linear array parallel to the short- or long-axis of the pill 621 (mono-plane, as shown in Figure 6(c)), a cross-patterned array 631 (bi-plane, as shown in Figure 6(d)), a linear array which can be rotated in an arrangement 641 (multi-plane, as shown in Figure 6(e)), a matrix array 651 (2-dimensional, as shown in Figure 6(f)), or along a ring surrounding the pill arrangement 661 (as shown in Figure 6(g)). The ultrasonic array can be further curved in any direction to provide a geometric focusing. In the exemplary transesophageal arrangement, an ultrasonic transducer can have a central frequency at, e.g., about 1 - 30 MHz.

The exemplary arrangement of other embodiments of the present disclosure can include a fluorescence imaging set-up configuration. For example, an exogenous or endogenous fluorophore in a tissue can be excited by a light at a wavelength λ₁, and can emit a second light at a red-shifted wavelength λ₂. Exemplary fluorophore can be selectively detected by selecting a proper combination of excitation and emission spectral bands. In one exemplary embodiment, the fluorescence signals can be emitted from vulnerable plaque, including the NIR region of the electromagnetic spectrum, which corresponds to emission from lipid oxidative byproducts. The intensity of the emission can also be a quantitative measure of the concentration of the fluorophore.

Exemplary fluorescence imaging procedure and arrangement according to an exemplary embodiment of the present disclosure is illustrated in Figure 7. For example, in a transesophageal pill endoscope arrangement 701, a light source 702 (e.g., a laser diode) can be provided which can emit an excitation at a wavelength λ₁, and a detector 703 (e.g., a photodiode) can measure an emitted light at a wavelength λ₂. A spectral filter 704 can be used to block unwanted light from reaching the detector 703. A balloon 705 can be attached to the pill endoscope arrangement 701, and can be inflated to anchor the arrangement 701 to one or more particular location to measure a tissue of interest. Furthermore, the fluorescence signal can be measured through a plurality of source-detector pairs, to provide a depth-resolved distribution of a fluorophore of interest using a reconstruction procedure (which is known to those having ordinary skill in the art) based on a light-tissue interaction model. It should be understood that other procedures and/or models that are known to those having ordinary skill in the art can be used with this exemplary embodiment, as well as with other exemplary embodiments that are described herein.

According to still another exemplary embodiment of the present disclosure, the exemplary arrangement can be configured to have an optical spectroscopy set-up configuration. For example, specific constituents of a tissue can have distinct optical spectra. It is possible to characterize the composition of the tissue, e.g., through a measurement of its optical spectra by decomposing the contributions from each constituent. An exemplary optical spectroscopy arrangement according to an exemplary embodiment of the present disclosure is illustrated in Figure 8. In a transesophageal pill endoscope 801 shown in Figure 8, a light source 802 (e.g., a superluminescent diode) can illuminate the tissue with a broadband light, and the reemitted light from the tissue can be measured by a spectrometer comprising a diffraction grating 803 and a camera 804 (e.g., a CCD or CMOS camera). A balloon 805 can be attached to the pill endoscope 801, and can be inflated to anchor such exemplary pill endoscope arrangement 801 to one or more particular locations to measure the tissue of interest. The optical spectra of the tissue, as shown in Figure 8, can be obtained by taking the ratio between the spectra of the excitation light and the emission light. A decomposing procedure may be used to estimate the concentration of a constituent of the tissue. It should be understood that other procedures and/or models that are known to those having ordinary skill in the art can be used with this exemplary embodiment, as well as with other exemplary embodiments that are described herein.

In yet another exemplary embodiment of the present disclosure, the exemplary arrangement/apparatus/endoscope can be provided in a laser speckle imaging set-up configuration. In such exemplary embodiment, e.g., the tissue is illuminated by a coherent light, a light scattered from the tissue can acquired by a camera showing a speckle pattern due to the interference. The spatial and/or temporal fluctuation(s) of the speckle can indicate the tissue perfusion and/or the mechanical properties of the tissue. The decorrelation time constant of the speckle intensity can be an index of viscoelasticity of a tissue that can be used to assess the structure and composition of an atherosclerotic plaque. (See Nadkami, S. K. et al., "Characterization of Atherosclerotic Plaques by Laser Speckle Imaging", Circulation 112, 885-892 (2005)).

An exemplary laser speckle imaging arrangement according to the exemplary embodiment of the present disclosure can include a transesophageal pill arrangement/endoscope, as illustrated in Figure 9. The exemplary pill arrangement 901 can include a miniaturized light source 902 (e.g., a microchip laser, or a laser diode) which can illuminate a tissue with a coherent optical wave, and an optical wave scattered by the tissue can be imaged by a camera 903 (e.g., a CCD or CMOS camera). An in-pill power unit/arrangement can energize both the source 902 and the camera 903. A balloon 904 can be attached to the pill arrangement 901, and can be inflated to anchor it to one or more particular locations, facilitating the ability by the camera 903 to obtain speckled images from the tissue of interest for a particular time period. In order to access a cardiac tissue or a blood vessel through an esophageal wall, an excitation light in the red and/or the near-infrared spectral range (e.g., 600 - 1100 nm) can be used. In addition, a spatial filter can be used to selectively detect light scattered from a deep tissue.

According to a further exemplary embodiment of the present disclosure, the exemplary arrangement/endoscope can be configured to have an optical tomography set-up configuration. In such exemplary embodiment, a plurality of optical sources and detectors can be provided, and a measurement of a diffused light reemitted from tissue can be performed following an optical illumination through, e.g., a plurality of source-detector pairs. A reconstruction procedure based on a light-tissue interaction model (known to those having ordinary skill in the art) can be employed to inverse the measurements so as to obtain optical properties of the tissue (e.g., an absorption coefficient, a reduced scattering coefficient, etc.), or a dynamic property of a tissue (e.g., blood flow). It should be understood that other procedures and/or models that are known to those having ordinary skill in the art can be used with this exemplary embodiment, as well as with other exemplary embodiments that are described herein.

An exemplary optical tomography imaging arrangement which is provided in a transesophageal pill arrangement configuration according to a further exemplary embodiment of the present disclosure is illustrated in Figure 10. For example, a pill endoscope/arrangement 1001 can include a plurality of sources 1002 (e.g., laser diodes) and a plurality of detectors 1003 (e.g., photodiodes). A balloon 1004 can be attached to the pill 1001, and can be inflated to anchor it to one or more particular locations when measuring or analyzing the tissue of interest. Another exemplary advantage for using the inflated balloon 1004 in a diffuse optical tomography apparatus can be that the balloon 1004 can impose well-defined boundary conditions to simplify the reconstruction. Sources 1002 can emit light in a sequential order (e.g., one source fires at a time), and the detectors 1003 can measure the resultant diffused reemission. The exemplary measurement can be transferred to an external computer, and reconstructed to obtain a property of the tissue.

According to another exemplary embodiment of the present disclosure, an ultrasonic imaging arrangement can be provided as illustrated in Figure 11. For example, in this exemplary embodiment, a wireless pill endoscope 1101 can be provided that is sized to fit in an esophagus, and the pill endoscope 1101 can include an ultrasonic transducer 1102 that can emit a high-frequency acoustic wave to insonify a tissue of a heart or a blood vessel, and detect a resultant acoustic echo scattered from the tissue. Due to the preference to penetrate an esophageal wall, the ultrasonic transducer 1102 can have a central frequency, e.g., between about 1 and 30 MHz. The detected acoustic signal can then be transferred to an external receiver, provided to a computer and analyzed to provide information about the tissue. The pill endoscope 1101 can include a balloon 1103, which can be inflated with an acoustic coupling medium (e.g., water) to stop at one or more particular positions to measure the tissue of interest. The exemplary ultrasonic transducer 1102 of the exemplary endoscope/apparatus 1101 can be used (e.g., with a computer commented thereto) the map an anatomy of the tissue based on their acoustic reflectivity, and can locate an atherosclerotic plaque by detect the thickening of a blood vessel wall. In addition, different subtypes of plaques can be differentiated by analyzing an acoustic signal. A fibrous plaque tends to give dense homogenous acoustic echo. Plaques with discrete lipid areas are hypoechogenic. Calcified plaques cast shadows behind hyperechogenic regions. Further, e.g., the exemplary ultrasonic set-up configuration shown in Figure 11 can measure the blood flow based on the Doppler principle. For example, by measuring a dimension of a heart or the blood vessel, and the blood velocity therein, the exemplary apparatus can further quantify a cardiac output or a cardiac index. Additionally, by detecting a deformation of the tissue after exerting a known pressure on it, a mechanic property of the tissue can be obtained.

A combination of two or more exemplary embodiments according to the present disclosure can provide complementary information about a cardiovascular disease or function, and is still inside the scope of the present disclosure. For example, with the exemplary photoacoustic arrangements illustrated in Figures 6(a)-6(g), the ultrasonic transducer 604 can be configured to emit an acoustic wave, detect the resultant acoustic echo and thus perform an additional ultrasonic imaging. The exemplary photoacoustic methods/procedures can be effective in obtaining functional and molecular information of the tissue, such as the molecular composition of a tissue, blood oxygenation, and temperature, while ultrasonic arrangement is good at mapping the structure of a tissue and measuring a blood flow. By implementing these exemplary procedures in a single probe, it is possible to use exemplary ultrasonic structural imaging procedure(s) to guide a placement of the photo-acoustic probe/endoscope/arrangement at one or more particular locations to measure a physiological function of the tissue of interest, obtain comprehensive information of an atherosclerotic plaque, obtain additional cardiac functional indicators, such an oxygen uptake by a lung, an oxygen delivery and consumption to different parts of the body, and/or assess to which extent a stenosis impedes oxygen delivery to the heart, etc.

An implementation of the exemplary embodiments of the methods and procedures according to the present disclosure discussed herein can increase the contrast of OCT and OFDI intracoronary images, thus possibly reducing the time and increasing the accuracy of interpreted images. Enhanced contrast and identification of areas with lipid can facilitate a rapid comprehensive visualization, and a guidance of local therapy methods and/or assessment of appropriate treatment options. This additional exemplary information on the tissue component, compound or chemical that is obtained by the disclosed method can be computed or determined using a processing apparatus (e.g., one or more computers), and displayed in real time in two dimensions or three dimensions to guide the exemplary diagnostic and/or therapeutic procedure.

It should be understood that for every exemplary embodiment described herein, any reference to optical spectroscopy can include diffuse optical tomography, optical coherence tomography, optical frequency domain imaging, and/or spectroscopic photoacoustics modalities. In addition, in or more exemplary embodiments described herein above, the exemplary catheters and/or endoscopes can be provided in various other vessels or orifices to measure different anatomical structures in proximity of the exemplary arrangements and/or structures. For example, the exemplary catheter(s)/arrangement(s)/endoscope(s) can be placed in the esophagus, and provided to measure a nearby anatomical structure, including the heart.

The foregoing merely illustrates the principles of the disclosure. Various modifications and alterations to the described embodiments will be apparent to those skilled in the art in view of the teachings herein. Indeed, the arrangements, systems and methods according to the exemplary embodiments of the present disclosure can be used with and/or implement any OCT system, OFDI system, SD-OCT system or other imaging systems, and for example with those described in International Patent Application PCT/US2004/029148, filed September 8, 2004 which published as International Patent Publication No. WO 2005/047813 on May 26, 2005, U.S. Patent Application No. 11/266,779, filed November 2, 2005 which published as U.S. Patent Publication No. 2006/0093276 on May 4, 2006, and U.S. Patent Application No. 10/501,276, filed July 9, 2004 which published as U.S. Patent Publication No. 2005/0018201 on January 27, 2005, and U.S. Patent Publication No. 2002/0122246, published on May 9, 2002. It will thus be appreciated that those skilled in the art will be able to devise numerous systems, arrangements and methods which, although not explicitly shown or described herein, embody the principles of the disclosure and are thus within the scope of the present disclosure. Further, the exemplary embodiments described herein can operate together with one another and interchangeably therewith.

## Claims

1. An apparatus for determining information regarding a tissue or an object at or within the tissue, comprising:
a pill endoscope comprising at least one first arrangement and at least one second arrangement and a spectrometer, the pill endoscope being configured to be situated inside a particular organ of a body, wherein the particular organ is an esophagus,
wherein the at least one first arrangement while being situated inside the particular organ is configured to cause a generation of at least one electromagnetic radiation in the tissue, wherein the tissue comprises (i) a heart, (ii) major vessels attached to the heart, (iii) coronary artery, and/or (iv) blood therein; and
wherein the at least one second arrangement is configured to:
(a) detect acoustic signals that are responsive to an interaction between the tissue and the at least one electromagnetic radiation, and
(b) measure or determine at least one of (i) at least one characteristic of the tissue, or (ii) information regarding the object at or in the tissue based on the detected acoustic signals;
wherein the spectrometer is configured to measure light reemitted from the tissue to determine an optical spectra of the tissue; and
at least one balloon arrangement which is configured to position the pill endoscope at a particular location within the particular organ.

2. The apparatus according to claim 1, wherein the at least one characteristic is a characteristic of at least one of the blood, the one or more major blood vessels coupled to the heart, or the one or more portions of the heart.

3. The apparatus according to claim 1, wherein the at least one second arrangement is further configured to determine further information to identify a possibility of coronary artery disease.

4. The apparatus according to claim 1, further comprising at least one of (i) at least one third arrangement which is configured to generate at least one image of the tissue as a function of the information, or (ii) at least one fourth arrangement which is configured to generate at least one image of the tissue and a further tissue that is in a proximity of the tissue.

5. The apparatus according to claim 4, wherein the at least one third arrangement is configured to obtain data for (i) a structure of the tissue, and (ii) the at least one characteristic approximately simultaneously.

6. The apparatus according to claim 5, wherein the data is at least one image of at least one of (i) the structure of the tissue, or (ii) the at least one characteristic superimposed on one another.

7. The apparatus according to claim 4, wherein the at least one fourth arrangement includes an ultrasound arrangement.

8. The apparatus according to claim 2, wherein the at least one second arrangement further measures at least one characteristic of at least one further tissue which includes one or more other tissues in addition to the at least one or more major blood vessel or portion of the heart.

9. The apparatus according to claim 1, wherein the pill endoscope is at least one of (i) structured and sized to be at least partially situated within the esophagus, or (ii) included with a transnasal device.

10. The apparatus according to claim 9, wherein the at least one characteristic is at least one of an oxygen saturation, a cardiac output, a blood flow, a total blood content, or a blood hematocrit.

11. The apparatus according to claim 10, wherein the oxygen saturation includes at least one of a venous oxygen saturation or an arterial oxygen saturation.

12. The apparatus according to claim 10, further comprising at least one third arrangement which is configured to generate at least one image of the anatomical structure as a function of the acoustic wave.

## Patentansprüche

1. Eine Vorrichtung zur Bestimmung von Informationen über ein Gewebe oder ein Objekt am oder im Gewebe, umfassend:
ein Pillenendoskop, das mindestens eine erste Anordnung und mindestens eine zweite Anordnung und ein Spektrometer umfasst, wobei das Pillenendoskop so ausgebildet ist, dass es innerhalb eines bestimmten Organs eines Körpers untergebracht ist, wobei das bestimmte Organ ein Ösophagus ist,
wobei die mindestens eine erste Anordnung, während sie sich innerhalb des bestimmten Organs befindet, so ausgebildet ist, dass sie eine Erzeugung von mindestens einer elektromagnetischen Strahlung in dem Gewebe bewirkt, wobei das Gewebe (i) ein Herz, (ii) mit dem Herz verbundene Hauptgefäße, (iii) eine Koronararterie, und/oder (iv) Blut darin umfasst; und
wobei die mindestens eine zweite Anordnung so ausgebildet ist, dass sie:
(a) akustische Signale, die auf eine Wechselwirkung zwischen dem Gewebe und der mindestens einen elektromagnetischen Strahlung reagieren, erfasst, und
(b) mindestens eines von (i) mindestens einer Eigenschaft des Gewebes, oder (ii) Informationen über das Objekt am oder im Gewebe auf Grundlage der erfassten akustischen Signale misst oder bestimmt;
wobei das Spektrometer so ausgebildet ist, dass es das von dem Gewebe reemittierte Licht misst, um ein optisches Spektrum des Gewebes zu bestimmen; und
mindestens eine Ballonanordnung, die so ausgebildet ist, dass sie das Pillenendoskop an einer bestimmten Stelle innerhalb des bestimmten Organs positioniert.

2. Die Vorrichtung nach Anspruch 1, wobei die mindestens eine Eigenschaft eine Eigenschaft von zumindest einem von: dem Blut, den einen oder mehreren Hauptblutgefäßen, die mit dem Herzen verbunden sind, oder der einen oder mehreren Teile des Herzens ist.

3. Die Vorrichtung nach Anspruch 1, wobei die mindestens eine zweite Anordnung ferner so ausgebildet ist, dass sie weitere Informationen zur Identifizierung einer Möglichkeit einer koronaren Herzkrankheit bestimmt.

4. Die Vorrichtung nach Anspruch 1, ferner umfassend mindestens eines von: (i) mindestens einer dritten Anordnung, die so ausgebildet ist, dass sie mindestens ein Bild des Gewebes in Abhängigkeit von der Information erzeugt, oder (ii) mindestens einer vierten Anordnung, die so ausgebildet ist, dass sie mindestens ein Bild des Gewebes und eines weiteren Gewebes, das sich in einer Nähe des Gewebes befindet, erzeugt.

5. Die Vorrichtung nach Anspruch 4, wobei die mindestens eine dritte Anordnung so ausgebildet ist, dass sie Daten für (i) eine Struktur des Gewebes und (ii) die mindestens eine Eigenschaft ungefähr gleichzeitig erhält.

6. Die Vorrichtung nach Anspruch 5, wobei es sich bei den Daten um mindestens ein Bild von mindestens einem von: (i) der Struktur des Gewebes oder (ii) der mindestens einen Eigenschaft, die einander überlagert sind, handelt.

7. Die Vorrichtung nach Anspruch 4, wobei die mindestens eine vierte Anordnung eine Ultraschallanordnung einschließt.

8. Die Vorrichtung nach Anspruch 2, wobei die mindestens eine zweite Anordnung ferner mindestens eine Eigenschaft mindestens eines weiteren Gewebes misst, das zusätzlich zu dem mindestens einen oder mehreren Hauptblutgefäß oder Teil des Herzens ein oder mehrere andere Gewebe einschließt.

9. Die Vorrichtung nach Anspruch 1, wobei das Pillenendoskop mindestens eines von: (i) so strukturiert und bemessen, dass es zumindest teilweise im Ösophagus untergebracht sein kann, oder (ii) in eine transnasale Vorrichtung integriert ist.

10. Die Vorrichtung nach Anspruch 9, wobei die mindestens eine Eigenschaft mindestens eines von: einer Sauerstoffsättigung, einer Herzleistung, einer Blutströmung, eines Gesamtblutgehalts, oder einem Bluthämatokrit ist.

11. Die Vorrichtung nach Anspruch 10, wobei die Sauerstoffsättigung mindestens eines von: einer venösen Sauerstoffsättigung oder einer arteriellen Sauerstoffsättigung einschließt.

12. Die Vorrichtung nach Anspruch 10, ferner umfassend mindestens eine dritte Anordnung, die so ausgebildet ist, dass sie mindestens ein Bild der anatomischen Struktur in Abhängigkeit von der akustischen Welle erzeugt.

## Revendications

1. Appareil pour déterminer des informations concernant un tissu ou un objet sur ou à l'intérieur du tissu, comportant :
un endoscope à capsule comportant au moins un premier agencement et au moins un deuxième agencement et un spectromètre, l'endoscope à capsule étant configuré pour être situé à l'intérieur d'un organe particulier d'un corps, dans lequel l'organe particulier est un oesophage,
dans lequel le au moins un premier agencement, tout en étant situé à l'intérieur de l'organe particulier, est configuré pour entraîner une génération d'au moins d'un rayonnement électromagnétique dans le tissu, dans lequel le tissu comporte (i) un coeur, (ii) des vaisseaux majeurs reliés au coeur, (iii) une artère coronaire et/ou (iv) du sang dans celui-ci ; et
dans lequel le au moins un deuxième agencement est configuré pour :
(a) détecter des signaux acoustiques qui sont sensibles à une interaction entre le tissu et le au moins un rayonnement électromagnétique, et
(b) mesurer ou déterminer au moins un élément parmi (i) au moins une caractéristique du tissu, ou (ii) des informations concernant l'objet sur ou dans le tissu sur la base des signaux acoustiques détectés ;
dans lequel le spectromètre est configuré pour mesurer une lumière réémise à partir du tissu pour déterminer un spectre optique du tissu ; et
au moins un agencement à ballonnet qui est configuré pour positionner l'endoscope à capsule à un emplacement particulier à l'intérieur de l'organe particulier.

2. Appareil selon la revendication 1, dans lequel la au moins une caractéristique est une caractéristique d'au moins un élément parmi le sang, le ou les vaisseaux sanguins majeurs couplés au coeur, et la ou les parties du coeur.

3. Appareil selon la revendication 1, dans lequel le au moins un deuxième agencement est en outre configuré pour déterminer des informations supplémentaires pour identifier une possibilité de coronaropathie.

4. Appareil selon la revendication 1, comportant en outre au moins un élément parmi (i) au moins un troisième agencement qui est configuré pour générer au moins une image du tissu en fonction des informations, ou (ii) au moins un quatrième agencement qui est configuré pour générer au moins une image du tissu et d'un tissu supplémentaire qui est à proximité du tissu.

5. Appareil selon la revendication 4, dans lequel le au moins un troisième agencement est configuré pour obtenir des données pour (i) une structure du tissu, et (ii) la au moins une caractéristique de manière sensiblement simultanée.

6. Appareil selon la revendication 5, dans lequel les données sont au moins une image d'au moins un élément parmi (i) la structure de tissu, ou (ii) la au moins une caractéristique, superposées l'une à l'autre.

7. Appareil selon la revendication 4, dans lequel le au moins un quatrième agencement inclut un agencement à ultrasons.

8. Appareil selon la revendication 2, dans lequel le au moins un deuxième agencement mesure en outre au moins une caractéristique d'au moins un tissu supplémentaire qui inclut un ou plusieurs autres tissus en plus du au moins un ou plusieurs vaisseaux sanguins majeurs ou parties du coeur.

9. Appareil selon la revendication 1, dans lequel l'endoscope à capsule est au moins (i) structuré et dimensionné pour être au moins en partie situé à l'intérieur de l'œsophage, ou (ii) inclus dans un dispositif transnasal.

10. Appareil selon la revendication 9, dans lequel la au moins une caractéristique est au moins un élément parmi une saturation en oxygène, un débit cardiaque, un débit sanguin, un taux sanguin total ou un hématocrite sanguin.

11. Appareil selon la revendication 10, dans lequel la saturation en oxygène inclut au moins un élément parmi une saturation en oxygène veineux ou une saturation en oxygène artériel.

12. Appareil selon la revendication 10, comprenant en outre au moins un troisième agencement qui est configuré pour générer au moins une image de la structure anatomique en fonction de l'onde acoustique.
